# FASCICULE DE BREVET EUROPEEN

(11) **EP 0 802 772 B1**
(45) Date de publication et mention de la délivrance du brevet: **21.10.1998**
(21) Numéro de dépôt: 96901378.8
(22) Date de dépôt: 11.01.1996
(51) Int. Cl.: A61B 17/70

(54) **FIXATEUR RACHIDIEN**
HALTEVORRICHTUNG FÜR DIE WIRBELSÄULE
SPINAL FIXATOR

(30) Priorité: 12.01.1995 FR 9500306
(43) Date de publication de la demande: 29.10.1997
(73) Titulaire: Euros, 13600 La Ciotat (FR); Gennari, Jean Marie, F-13008 Marseille (FR)
(72) Inventeur: GENNARI, Jean-Marie, Les Jardins de Thalassa, B, F-13008 Marseille (FR)
(74) Mandataire: Texier, Christian
(86) Numéro de dépôt international: FR9600046
(87) Numéro de publication internationale: WO9621396

(56) Documents cités:
- EP-A- 0 465 158
- EP-A- 0 577 219
- DE-U- 9 403 231
- US-A- 5 020 519

## Description

La présente invention concerne un nouveau type d'implant rachidien. Plus particulièrement, elle concerne un implant rachidien du type qui comporte des moyens de fixation à une vertèbre, une tige de liaison, une partie de montage s'étendant sur un axe X-X' et comprenant un logement ouvert du côté opposé aux moyens de fixation et comportant des ouvertures latérales opposées pour recevoir une tige de liaison s'étendant transversalement l'axe X-X' de part et d'autre de ladite partie de montage pour être solidarisée avec au moins un implant rachidien, un élément de verrouillage destiné à coopérer avec ladite partie de montage pour bloquer ladite tige de liaison à l'intérieur dudit logement et une bague intermédiaire de forme symétrique de révolution autour de l'axe X-X'.

L'invention concerne également un appareil d'assemblage pour un tel implant rachidien.

On connaît déjà un implant rachidien de ce type, dans lequel l'élément de verrouillage est une vis apte à être vissée dans un taraudage prévu dans le logement de la partie de montage de l'implant rachidien, en vue de bloquer la tige qui est prépositionnée manuellement dans ledit logement.

Ce type d'implant assemblé avec une tige de liaison est utilisé pour traiter des arthroses, des fractures vertébrales ou pour corriger des déviations de la colonne vertébrale telles qu'une scoliose ou une cyphose.

Le chirurgien fixe en général deux implants par vertèbre, puis positionne dans chacun des deux implants une tige moletée, ces deux tiges étant parallèles et préalablement cintrées en fonction du traitement et de la correction à apporter sur la colonne vertébrale.

Cependant, l'inconvénient principal de cet implant de type connu est que la mise en place de la vis de verrouillage dans le logement de la partie de montage munie de la tige plus ou moins cintrée, est souvent une opération difficile et délicate à réaliser.

En effet, lorsque la tige moletée plus ou moins cintrée est positionnée à l'intérieur du logement de la partie de montage de cet implant, elle a tendance à remonter pour sortir du logement en bloquant l'accès de l'élément de verrouillage au taraudage du logement. Le chirurgien doit alors appuyer sur ladite tige à l'aide de l'élément de verrouillage tout en vissant celui-ci à l'intérieur du logement, pour obliger celle-ci à se positionner dans le logement et la bloquer à l'intérieur de ce dernier à l'aide de l'élément de verrouillage. Dans certains cas, lorsque la tige présente un cintrage accentué, il se peut que lors de la mise en place de l'élément de verrouillage à l'intérieur de logement de la partie de montage de l'implant, celle-ci se casse.

Par ailleurs, on connaît du document DE-U-94032319, un implant qui comporte une vis de fixation à une vertèbre, une plate-forme de montage qui s'étend transversalement à l'axe de la vis de fixation à l'opposé de celle-ci. Les deux parois de montage définissent entre elles un logement ouvert du côté opposé à la vis de fixation et comportant des ouvertures latérales pour recevoir une tige de liaison s'étendant selon l'axe de la plate-forme de montage de part et d'autre dudit logement. L'implant comporte en outre un élément intermédiaire de montage qui comprend une partie supérieure en forme de manchon cylindrique munie d'un filetage interne dans lequel est vissé un élément de verrouillage pour bloquer la tige de liaison sur la plate-forme de montage. L'élément intermédiaire de montage comporte une partie inférieure constituée par deux parois latérales s'étendant en direction de la vis de fixation, et portant chacune à leur extrémité libre une patte de fixation munie d'une dent qui s'étend selon la direction circonférentielle de la partie supérieure dudit élément intermédiaire de montage. L'élément intermédiaire de montage est mis en place par rotation sur les parois en vis-à-vis de la plate-forme de montage, jusqu'à ce que ses pattes de fixation portant les dents viennent se positionner sous la plate-forme de montage et coopèrent avec un logement concave prévu sous cette dernière. Dans la position assemblée, l'élément intermédiaire de montage ne prend pas appui sur la tige de liaison et ne bloque pas celle-ci en translation selon l'axe de ladite tige dans son logement.

Enfin, on connaît du document EP. 465 158 un implant dont les caractéristiques correspondent à celles énoncées dans le préambule de la revendication 1. Selon le document EP. 465 158, la bague intermédiaire est un simple manchon mis en place par coulissement sur la partie de montage simultanément avec l'élément de verrouillage qui est vissé à l'intérieur de la partie de montage. Lorsque l'élément de verrouillage est totalement vissé, la bague prend appui sur la tige de liaison et la bloque à l'intérieur du logement de la partie de montage.

Afin de pallier les inconvénients de l'état de la technique précitée, l'invention propose un nouveau implant rachidien qui permet un pré-serrage et un prépositionnement de la tige à l'intérieur du logement de la partie de montage de l'implant, ce qui permet de dégager la partie du logement apte à recevoir l'élément de verrouillage et autorise une mise en place aisée de l'élément de verrouillage dans ledit logement tout en gardant un encombrement minimum dudit implant.

Plus particulièrement, dans l'implant rachidien selon l'invention ladite bague intermédiaire est une bague de pré-serrage destinée à être positionnée sur la partie de montage avant la mise en place de l'élément de verrouillage, ladite bague intermédiaire de pré-serrage coopérant par un encliquetage à déformation élastique avec la partie de montage et venant en appui contre ladite tige de liaison pour prépositionner cette dernière dans ledit logement et faciliter la mise en place ultérieure de l'élément de verrouillage dans ce dernier quel que soit le cintrage de ladite tige de liaison.

Selon un mode réalisation préféré de l'implant rachidien selon l'invention, ladite bague intermédiaire de pré-serrage est apte à être montée sur la surface externe de ladite partie de montage, et comprend deux bords d'appui en forme d'arche aptes à s'appuyer sur la tige de liaison de part et d'autre de la partie de montage, lorsque ladite bague est encliquetée avec ladite partie de montage.

Selon une caractéristique avantageuse de l'implant rachidien conforme à l'invention, la bague intermédiaire de pré-serrage présentant comme axe de symétrie l'axe X-X', comporte de part et d'autre de chaque bord d'appui en forme d'arche deux fentes qui s'étendent sensiblement parallèlement à l'axe X-X' et qui confèrent à chaque bord d'appui une souplesse d'appui sur ladite tige de liaison.

Par ailleurs, l'appareil d'assemblage conforme à l'invention, comporte une première pince apte à serrer entre ses branches la base de la partie de montage dudit implant rachidien et une deuxième pince comprenant à une extrémité une patte de forme générale en U apte à se placer à cheval sur la tige de liaison pour la positionner en regard du logement de la partie de montage de l'implant et un manchon destiné à supporter la bague intermédiaire de pré-serrage et déplaçable à coulissement sur ladite patte pour encliqueter celle-ci avec la partie de montage de l'implant et pré-positionner ladite tige de liaison dans le logement, la première pince et la deuxième pince comportant chacune une butée, lesdites butées étant destinées à venir en appui mutuel lors de l'assemblage de l'implant rachidien avec la tige de liaison de façon à reprendre les efforts de poussée exercés sur l'implant par de la deuxième pince lors de l'encliquetage de la bague intermédiaire de serrage avec la partie de montage de l'implant.

La description qui va suivre en regard des dessins annexés, donnés à titre d'exemples non limitatifs, fera bien comprendre en quoi consiste l'invention et comment elle peut être réalisée.

Sur les dessins annexés :
- la figure 1 est une vue schématique en perspective de trois implants rachidiens conformes à l'invention assemblés avec une tige de liaison, un desdits implants rachidiens étant représenté en vue éclatée,
- les figures 2a à 2b représentent en vue schématique en coupe longitudinale, les différentes étapes d'assemblage d'un implant rachidien conforme à l'invention avec une tige de liaison,
- la figure 3 représente une vue schématique en perspective de l'appareil d'assemblage d'un implant rachidien conforme à l'invention avec une tige de liaison,
- la figure 4 représente une vue en perspective de la deuxième pince de l'appareil d'assemblage de la figure 3,
- la figure 5 est une vue agrandie de détails d'une extrémité de la deuxième pince représentée sur la figure 4,
- la figure 6 représente est vue schématique en perspective de l'appareil d'assemblage de la figure 3 avec la deuxième pince à cheval sur la tige de liaison positionnée en regard du logement de la partie de montage de l'implant rachidien conforme à l'invention,
- la figure 7 représente une vue schématique en perspective de l'appareil d'assemblage de la figure 3 , dans laquelle la deuxième pince encliquete la bague intermédiaire de pré-serrage sur la partie de montage de l'implant rachidien conforme à la mention.

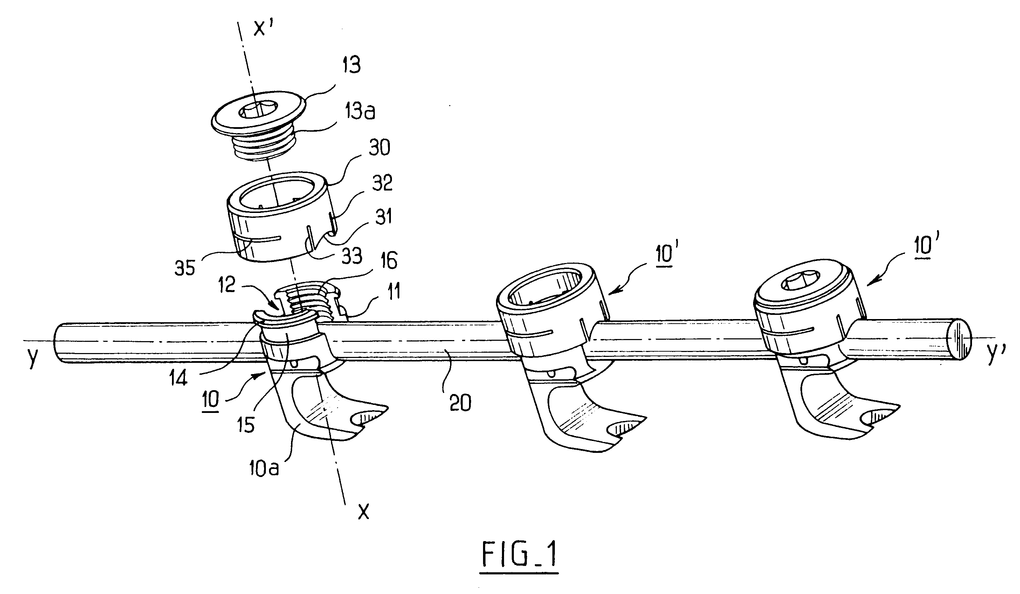

Sur la figure 1 on a représenté un implant rachidien qui comporte des moyens de fixation à une vertèbre. Selon le mode de réalisation représenté ces moyens de fixation sont constitués par un crochet 10a apte à s'accrocher sur une vertèbre. Bien entendu, on peut envisager selon un autre mode de réalisation ici non représenté que ces moyens de fixation comprennent une tige filetée permettant de visser l'implant rachidien sur la vertèbre.

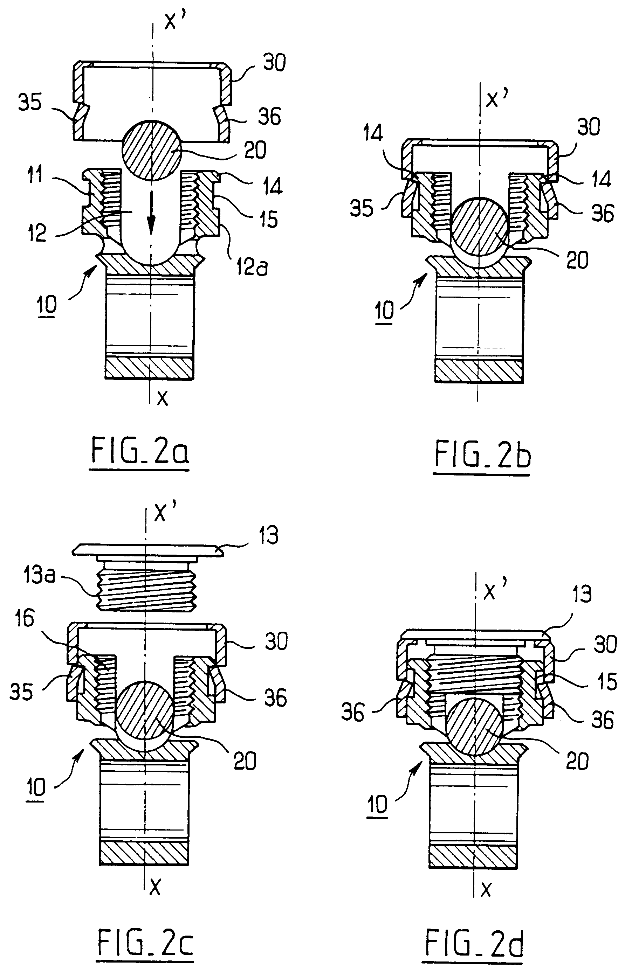

On distingue sur la figure 1 que l'implant rachidien 10 comporte une partie de montage 11 dont la base est solidaire du crochet de fixation 10a. Cette partie de montage 11 s'étend selon un axe longitudinal X-X', et présente une forme générale sensiblement cylindrique de révolution autour de cet axe longitudinal X-X' . La partie de montage 11 comporte un logement 12 muni d'une ouverture située du côté opposé au crochet de montage 10a et deux autres ouvertures latérales opposées de façon symétrique par rapport à l'axe X-X'. Vu en coupe, comme représenté sur les figures 2a à 2d, le logement 12 présente une forme sensiblement en U. Le logement 12 est destiné à recevoir une tige de liaison moletée 20 cintrée ou non, s'étendant selon une direction Y-Y' transversale à l'axe X-X', de part et d'autre de la partie de montage 11 pour être solidarisée avec ici deux autres implants rachidiens 10'. La paroi interne du logement 12 est pourvue en partie supérieure à proximité de son ouverture située côté opposé au crochet de fixation 10a, un taraudage 16 apte à coopérer avec une visse de verrouillage 13 comprenant un filetage 13a apte à être engagé dans ledit logement afin de bloquer la tige de liaison 20 dans le fond du logement 12.

Par ailleurs l'implant rachidien 10 comporte une bague intermédiaire de pré-serrage 30 qui présente une forme symétrique de révolution autour de l'axe X-X' et qui est apte à coopérer par encliquetage avec la partie de montage 11 pour venir en appui contre la tige de liaison 20 afin de la pré- positionner dans le logement 12 (voir figure 2b).

Plus particulièrement cette bague de pré-serrage 30 comporte deux bords d'appui 31 formés dans le bord inférieur périphérique de ladite bague et disposés de manière opposée symétriquement par rapport à l'axe X-X'. Ces deux bords d'appui 31 présentent une forme en arche et sont aptes à s'appuyer comme le montre la figure 1 sur la surface externe de la tige de liaison 20, de part et d'autre de la partie de montage 11, lorsque la bague 30 est encliquetée avec la partie de montage 11. Cette bague intermédiaire 30 est bien entendue positionnée sur la partie de montage 11 avant le vissage de la vis de verrouillage 13 à l'intérieur du logement 12 pour bloquer définitivement la tige de liaison 20 dans le logement 12. Cette bague intermédiaire de pré-serrage permet de pré-positionner ladite tige 20 dans le logement 12, en dégageant le taraudage 16 dudit logement 12 de façon à faciliter la mise en place de l'élément de verrouillage 13 dans le logement 12. A cet effet il est intéressant de préciser, comme le montre plus particulièrement les figures 2b et 2c, que lorsque la bague intermédiaire de pré-serrage 30 est mise en place sur la partie du montage en appui sur la tige de liaison 20, celle-ci n'est pas positionnée contre le fond du logement 12 ce qui permet d'ajuster la position relative des vertèbres et de la tige de liaison par coulissement et/ou rotation de la tige dans les différents implants ou bien par coulissement et/ou rotation d'un implant sur la tige de liaison.

Une telle structure offre de nombreux avantages en chirurgie du rachis et permet notamment de faciliter grandement l'opération de réduction d'une vertèbre.

Comme le montre les figures 2a à 2d, la bague intermédiaire de pré-serrage 30 comporte dans sa paroi latérale deux languettes d'encliquetage 35,36 positionnées symétriquement par rapport a l'axe X-X' entre les bords d'appui 31 en forme d'arche.

Ces languettes d'encliquetage 35,36 comme le montre plus particulièrement la figure 1 s'étendent suivant un arc de cercle. Elles sont aptes lors de l'encliquetage de la bague intermédiaire de pré-serrage sur la partie de montage, à se déformer élastiquement en coopérant avec un rebord annulaire 14 de la partie de montage pour venir en butée contre ce rebord 14. Ce rebord annulaire 14 prévu sur la surface externe de la partie de montage 11, constitue un rebord supérieur d'une gorge annulaire 15. Comme le montre les figures 2c et 2d lorsque la vis de verrouillage 13 est vissée à l'intérieur du logement 12 de la partie de montage 11 de l'implant 10, celle-ci enfonce la bague intermédiaire de pré-serrage selon la direction X-X', de telle sorte que les languettes d'encliquetage 35,36 glissent sur la paroi de la gorge 15 jusqu'à se placer dans une position de blocage où la vis de verrouillage 13 est en appui contre la tige moletée 20 positionnée sur le fond du logement 12.

Selon une caractéristique avantageuse de la bague intermédiaire de pré-serrage 30, on remarquera sur la figure 1 que celle-ci comporte de part et d'autre de chaque bord d'appui en forme d'arche 31, des fentes 33,32 s'étendant parallèlement à l'axe X-X' et conférant à ce dernier une souplesse d'appui.

Plus particulièrement, lors de l'encliquetage de la bague intermédiaire de pré-serrage 30 sur la partie de montage 11, les fentes 32,33 se positionnent de façon sensiblement perpendiculaire à la surface externe de la tige de liaison 20 pour permettre un appui optimal de chaque bord d'appui 31 contre la tige de liaison 20. Ce positionnement des fentes perpendiculaire à la surface externe de la tige 20 est réalisé quel que soit le cintrage de la tige 20.

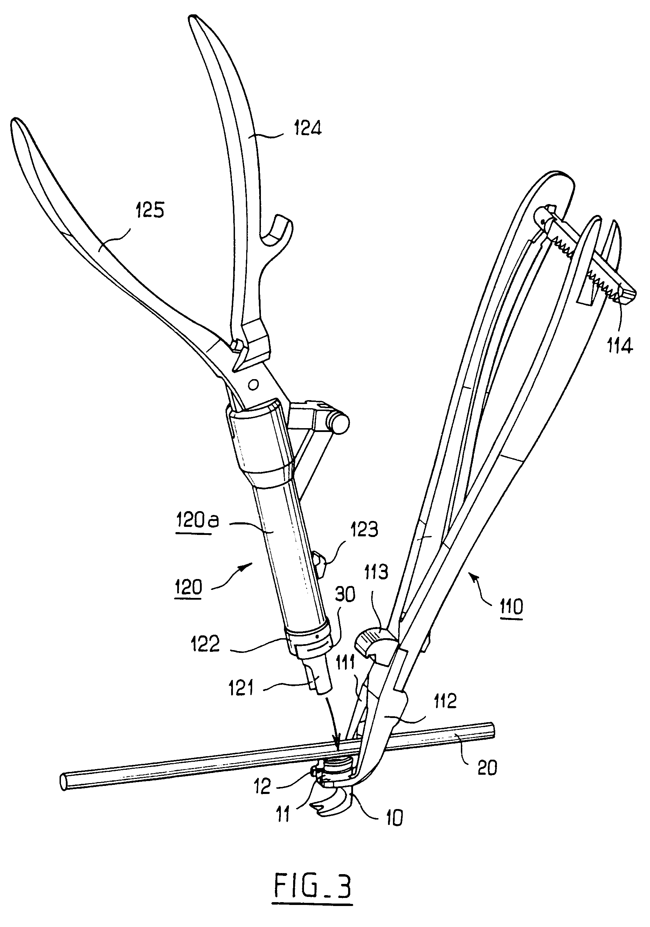

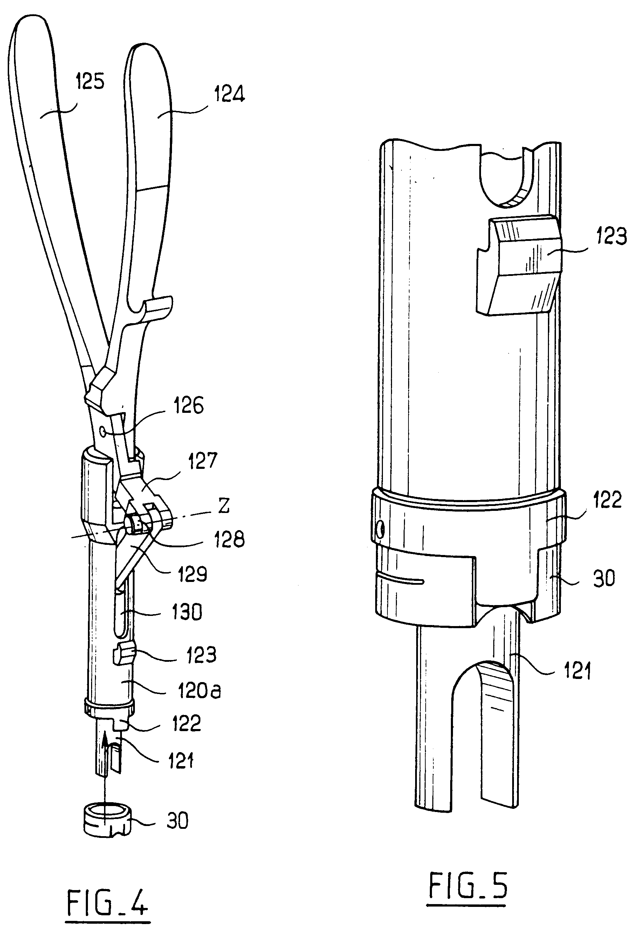

En référence aux figures 3 à 7, nous allons maintenant décrire l'appareil d'assemblage conforme à l'invention, qui permet d'assembler un implant rachidien du type représenté sur la figure 1 avec une tige de liaison moletée.

Cet appareil d'assemblage comporte une première pince 110 munie de deux branches 111,112 articulées autour d'un axe (ici non représenté) et comprenant des leviers d'actionnement desdites branches s'étendant dans le prolongement de chacune des branches . Les branches 111,112 sont aptes à maintenir serrée la base, de la partie de montage 11 de l'implant 10. On remarquera qu' à l'extrémité des leviers d'actionnement des branches la première pince 110 est pourvue d'un système de blocage 14 à crémaillère qui permet de bloquer la pince dans une position donnée de serrage dudit implant. En outre, la première pince 110 comporte au niveau de l'articulation desdites branches 111, 112 une butée 113. Ainsi, pour assembler la tige de liaison 20 avec l'implant 10 le chirurgien sert la base de la partie de montage de l'implant avec cette première pince 110 qu'il bloque en position de serrage.

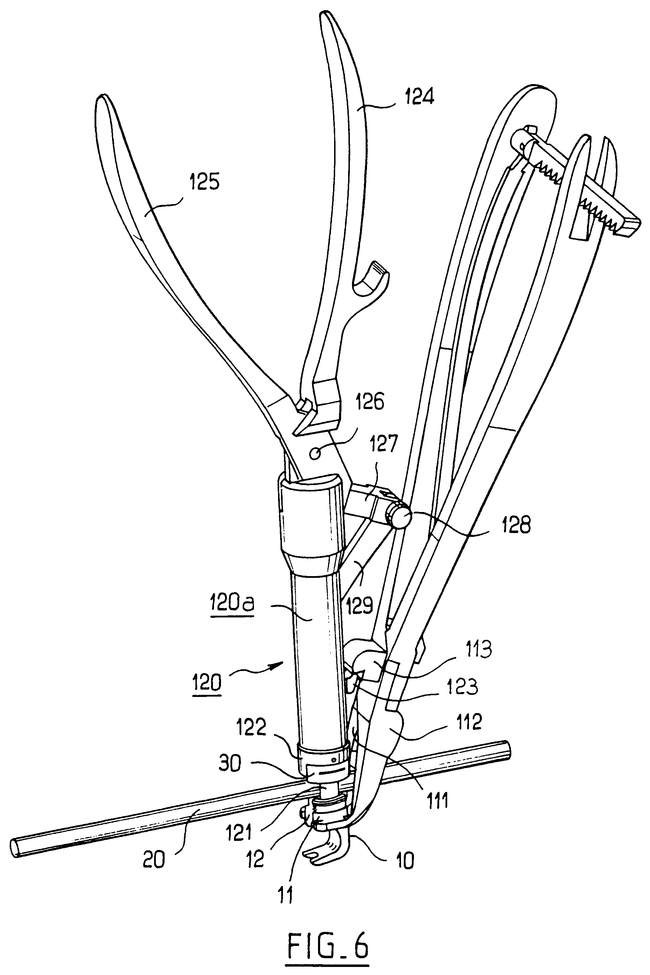

En outre l'appareil d'assemblage comporte une deuxième pince 120 munie d'un corps tubulaire 120, et a une extrémité de ce corps, une patte 121 de forme générale en U apte à se placer à cheval sur la tige de liaison 20 pour la positionner en regard du logement 12 de la partie de montage 11 (voir figure 6). Cette deuxième pince 120 comporte un manchon 122 solidaire d'une tige 130 montée à coulissement à l'intérieur du corps tubulaire 120 et actionnable à l'aide de deux leviers 124,125 pivotants autour d'un axe 126 et le levier 125 étant relié à un système de bielles articulées 127,129, la bielle 129 étant elle-même reliée à ladite tige 130. Ce manchon 122 est destiné à supporter la bague intermédiaire de pré-serrage 30. Par l'intermédiaire de la tige 130 coulissante à l'intérieur du corps tubulaire 120, le manchon 122 est donc déplaçable à coulissement sur la patte 121 en forme de U.

Plus particulièrement, en rapprochant les leviers d'actionnement 124, 125 de ladite deuxième pince 120 on déplace la tige 130 qui entraîne en coulissement sur la patte 121 le manchon 122 comportant la bague intermédiaire de pré-serrage 30 de sorte que la bague de pré-serrage vient en appui sur la tige de liaison 20 préalablement positionnée en regard du logement 12 et de la partie de montage 11, puis en continuant à faire coulisser le manchon 122 en direction de la partie de montage 10, le manchon 122 encliquette la bague de pré-serrage 30 sur la partie de montage 11 en pré-positionnant la tige moletée de liaison 20 à l'intérieur du logement 12. Il convient de noter que l'engagement de la tige de liaison 20 l'intérieur du logement 12 s'effectue simultanément à l'encliquetage de la bague intermédiaire de pré-serrage 30 sur la partie de montage 11 de l'implant 10.

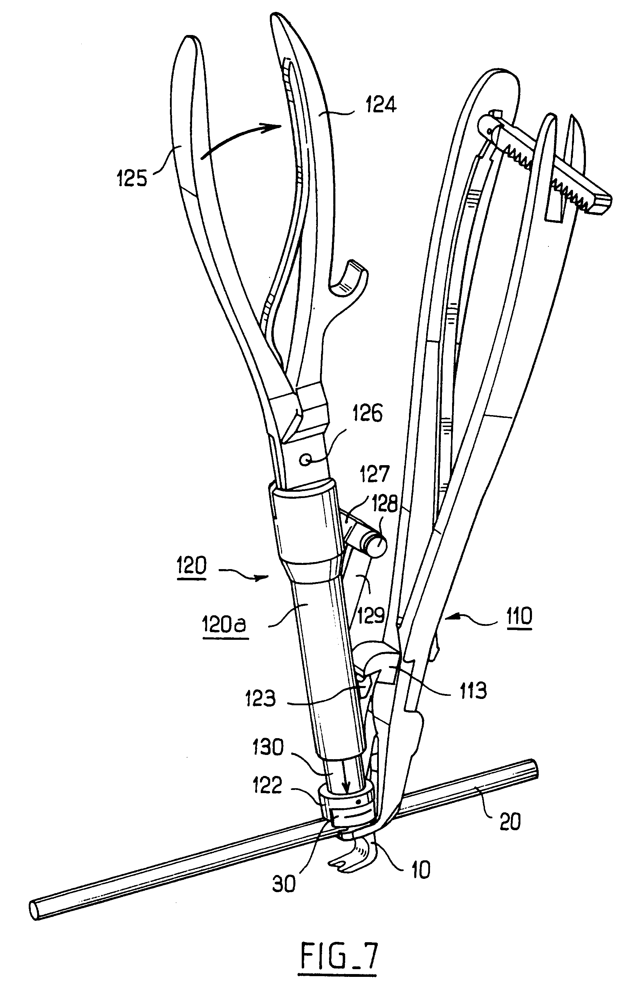

Selon une caractéristique avantageuse de la deuxième pince 120 celle-ci comporte sur la paroi externe de son corps tubulaire 120a une butée 123. Cette butée 123 est destinée à venir en appui contre la butée 113 de la première pince 110 lors de l'assemblage de l'implant 10 avec la tige de liaison 20 (voir figures 6 et 7) de sorte que ces butées 113, 123 reprennent les efforts de poussée exercés sur l'implant 10 par la deuxième pince 120 au cours de l'encliquetage de la bague intermédiaire de serrage 30 avec la partie de montage 11 de l'implant 10.

La présente invention n'est nullement limitée au mode de réalisation décrit et représenté mais l'homme du métier saura envisager tout variante conforme à son esprit.

Par exemple , on peut envisager selon une variante non représentée que la bague de préserrage soit montée dans le logement de la partie de montage de l'implant, la vis de verrouillage venant alors se visser sur la partie de montage pour enfoncer ladite bague de pré-serrage dans le logement et bloquer ainsi la tige de liaison.

## Revendications

1. Implant rachidien (10) qui comporte des moyens de fixation à une vertèbre, une tige de liaison (20), une partie de montage (11) s'étendant selon un axe X-X' et comprenant un logement (12) ouvert du côté opposé aux moyens de fixation et comportant des ouvertures latérales opposées pour recevoir la tige de liaison (20) s'étendant transversalement à l'axe X-X' de part et d'autre de ladite partie de montage (11) pour être solidarisée avec au moins un autre implant rachidien (10'), un élément de verrouillage (13) destiné à coopérer avec ladite partie de montage (11) pour bloquer ladite tige de liaison (20) à l'intérieur dudit logement (12) et une bague intermédiaire (30) de forme symétrique de révolution autour de l'axe X-X', caractérisé en ce que ladite bague intermédiaire (30) est une bague de pré-serrage destinée à être positionnée sur la partie de montage (11) avant la mise en place de l'élément de verrouillage (13), ladite bague intermédiaire de pré-serrage (30) coopérant par un encliquetage à déformation élastique avec la partie de montage (11) et venant en appui contre ladite tige de liaison (20) pour prépositionner cette dernière dans ledit logement (12) et faciliter la mise en place de l'élément de verrouillage (13) dans ce dernier quel que soit le cintrage de ladite tige de liaison.

2. Implant rachidien (10) selon la revendication 1, caractérisé en ce que ladite bague intermédiaire de pré-serrage (30) étant apte à être montée sur la surface externe (15) de ladite partie de montage (11), elle comprend deux bords d'appui (31) en forme d'arche aptes à s'appuyer sur la tige de liaison (20) de part et d'autre de la partie de montage (11) lorsque ladite bague (30) est encliquetée avec ladite partie de montage.

3. Implant rachidien (10) selon la revendication 2, caractérisé en ce que la bague intermédiaire de pré-serrage (30) présentant comme axe de symétrie l'axe X-X', comporte de part et d'autre de chaque bord d'appui en forme d'arche (31) deux fentes (32,33) qui s'étendent sensiblement parallèlement à l'axe X-X' et qui conférent à chaque bord d'appui (31) une souplesse d'appui sur ladite tige de liaison (20).

4. Implant rachidien (10) selon la revendication 3, caractérisé en ce que lesdites fentes (32,33) prévues de part et d'autre de chaque bord d'appui (31) sont aptes, lors de l'encliquetage de ladite bague intermédiaire de pré-serrage (30) avec ladite partie de montage (11), à se positionner sensiblement perpendiculairement à la surface externe de la tige de liaison (20) pour permettre un appui optimal de chaque bord d'appui en forme d'arche (31) contre ladite tige de liaison (20).

5. Implant rachidien (10) selon l'une quelconque des revendications 1 à 4, caractérisé en ce que la bague intermédiaire de pré-serrage (30) comporte deux languettes d'encliquetage (35,36) disposées symétriquement par rapport à l'axe X-X' entre lesdits bords d'appui (31), ces deux languettes d'encliquetage (35,36) étant aptes, lors du montage de la bague intermédiaire de pré-serrage (30) sur la partie de montage (11) de l'implant (10), à se déformer élastiquement pour venir en butée contre un rebord (14) prévu sur la partie de montage (11).

6. Implant rachidien (10) selon l'une quelconque des revendications 2 à 5, caractérisé en ce que le logement (12) de ladite partie de montage (11) est pourvu en partie supérieure d'un taraudage (16) destiné à coopérer avec l'élément de verrrouillage (13) fileté engagé dans ledit logement (12) la bague de pré-serrage (30) étant apte à prépositionner ladite tige de liaison (20) dans ledit logement (12), de telle sorte que la taraudage (16)soit dégagé pour le vissage de l'élément de verrouillage (13).

7. Appareil d'assemblage (100) pour un implant rachidien (10) selon l'une quelconque des revendications 1 à 6, caractérisé en ce qu'il comporte une première pince (110) apte à serrer entre ses branches (111,112) la base de la partie de montage (11) dudit implant rachidien (10), et une deuxième pince (120) comprenant à une extrémité une patte (121) de forme générale en U apte à se placer à cheval sur la tige de liaison (20) pour la positionner en regard du logement (12) de la partie de montage (11) de l'implant (10), et un manchon (122) déplaçable à coulissement sur ladite patte (121) et destiné à supporter la bague intermédiaire de pré-serrage (30), pour encliqueter celle-ci par déformation élastique avec la partie de montage (11) de l'implant (10) et prépositionner ladite tige de liaison (20) dans le logement (12), la première pince (110) et la deuxième pince (120) comportant chacune une butée (113,123) lesdites butées (113, 123) étant destinées à venir en appui mutuel lors de l'assemblage de l'implant rachidien (10) de façon à reprendre les efforts de poussée exercés sur l'implant (10) par la deuxième pince (120) lors de l'encliquetage par déformation élastique de la bague intermédiaire de serrage (30) avec la partie de montage (11) de l'implant (10).

## Claims

1. A spinal implant (10) which includes means for fixing it to a vertebra, a connecting rod (20), a mounting part (11) extending along an axis X-X' and comprising a housing (12) open on the side opposite the fixing means and including opposed lateral openings to receive the connecting rod (20) extending transversely to the axis X-X' on either side of said mounting part (11) to be fastened to at least one other spinal implant (10'), a locking member (13) adapted to cooperate with said mounting part (11) to immobilise said connecting rod (20) inside said housing (12) and a symmetrical circular intermediate ring (30) coaxial with the axis X-X', characterised in that said intermediate ring (30) is a pre-clamping ring adapted to be fitted to the mounting part (11) before fitting the locking member (13), said pre-clamping intermediate ring (30) cooperating with the mounting part (11) by clipping to it by virtue of elastic deformation and bearing against said connecting rod (20) to preposition the latter in said housing (12) and facilitate the fitting of the locking member (13) into the latter regardless of the curvature of said connecting rod.

2. A spinal implant (10) according to claim 1 characterised in that, said pre-clamping intermediate ring (30) being adapted to be mounted on the outside surface (15) of said mounting part (11), it comprises two arcuate bearing edges (31) adapted to bear on the connecting rod (20) on either side of the mounting part (11) when said ring (30) is clipped to said mounting part.

3. A spinal implant (10) according to claim 2 characterised in that the pre-clamping intermediate ring (30), being symmetrical about the X-X' axis, includes on either side of each arcuate bearing edge (31) two slots (32, 33) extending substantially parallel to the axis X-X' and which confer upon each bearing edge (31) flexibility for bearing engagement with said connecting rod (20).

4. A spinal implant (10) according to claim 3 characterised in that said slots (32, 33) on either side of each bearing edge (31) are adapted, when said pre-clamping intermediate ring (30) is clipped to said mounting part (11), to assume a position substantially perpendicular to the outside surface of the connecting rod (20) to enable optimal bearing of each arcuate bearing edge (31) on said connecting rod (20).

5. A spinal implant (10) according to any one of claims 1 to 4 characterised in that the pre-clamping intermediate ring (30) includes two clipping tongues (35, 36) disposed symmetrically about the axis X-X' between said bearing edges (31), these two clipping tongues (35, 36) being adapted, during mounting of the pre-clamping intermediate ring (30) on the mounting part (11) of the implant (10), to deform elastically to abut against a rim (14) on the mounting part (11).

6. A spinal implant (10) according to any one of claims 2 to 5 characterised in that the housing (12) of said mounting part (11) has a screwthread (16) at the top adapted to cooperate with the screwthreaded locking member (13) engaged in said housing (12), the pre-clamping ring (30) being adapted to preposition said connecting rod (20) in said housing (12) so that the screwthread (16) is uncovered for screwing the locking member (13).

7. Apparatus (100) for assembling a spinal implant (10) according to any one of claims 1 to 6 characterised in that it includes a first clamp (110) adapted to clamp the base of the mounting part (11) of said spinal implant (10) between its branches (111, 112) and a second clamp (120) comprising at one end a generally U-shaped lug (121) adapted to straddle the connecting rod (20) to position it facing the housing (12) of the mounting part (11) of the implant (10) and a sleeve (122) adapted to slide on said lug (121) and to support the pre-clamping intermediate ring (30) for clipping the latter by elastic deformation to the mounting part (11) of the implant (10) and prepositioning said connecting rod (20) in the housing (12), the first clamp (110) and the second clamp (120) each including an abutment (113, 123), said abutments (113, 123) being adapted to bear on each other during assembly of the spinal implant (10) so as to absorb thrust forces exerted on the implant (10) by the second clamp (120) during clipping of the clamping intermediate ring (30) to the mounting part (11) of the implant (10) by elastic deformation.

## Patentansprüche

1. Haltevorrichtung (10) für die Wirbelsäule, welche Vorrichtung Mittel zur Befestigung an einem Wirbel umfaßt, einen Verbindungsstab (20), ein Montagestück (11), das sich in Richtung einer Achse X-X' erstreckt und einen Sitz (12) aufweist, der zu der den Befestigungsmitteln abgewandten Seite hin offen ist und einander gegenüberliegende seitliche Öffnungen zur Aufnahme des Verbindungsstabes (20) aufweist, der quer zu der Achse X-X' von einer Seite des genannten Montagestücks (11) zur anderen verläuft, um eine starre Verbindung mit mindestens einer weiteren Haltevorrichtung (10') für die Wirbelsäule herzustellen, ferner ein Verriegelungselement (13), das mit dem genannten Montagestück (11) zusammenwirken und den genannten Verbindungsstab (20) innerhalb des genannten Sitzes (12) blockieren soll, sowie einen bezüglich der Achse X-X' rotationssymmetrischen Zwischenring (30),
dadurch gekennzeichnet, daß der genannte Zwischenring (30) einen vorläufig fixierenden Ring bildet, der vor dem Anbringen des Verriegelungselements (13) auf das Montagestück (11) aufgesetzt werden soll, welcher vorläufig fixierende Zwischenring (30) durch Vermittlung eines elastisch verformbaren Rastelements mit dem Montagestück (11) zusammenwirkt und sich an den genannten Verbindungsstab (20) anlegt, um diesen in dem genannten Sitz (12) vorläufig zu fixieren und das Anbringen des Verriegelungselements (13) in diesem zu erleichtern, unabhängig von der Biegung des genannten Verbindungsstabes.

2. Haltevorrichtung (10) für die Wirbelsäule, nach Anspruch 1, dadurch gekennzeichnet, daß der genannte vorläufig fixierende Zwischenring (30), der auf die Außenfläche (15) des genannten Montagestücks (11) gesetzt werden kann, zwei bogenförmige Auflageränder (31) aufweist, die sich beiderseits des Montatgestücks (11) auf dem Verbindungsstab (20) abzustützen vermögen, wenn der genannte Ring (30) mit dem genannten Montageteil verrastet wird.

3. Haltevorrichtung (10) für die Wirbelsäule, nach Anspruch 2, dadurch gekennzeichnet, daß der die Achse X-X' als Symmetrieachse aufweisende vorläufig fixierende Zwischenring (30) beiderseits jeder bogenförmigen Stützkante (31) jeweils einen von zwei im wesentlichen parallel zu der Achse X-X' verlaufenden Schlitzen (32,33) aufweist, die jeder Stützkante (31) eine nachgiebige Abstützung auf dem genannten Verbindungsstab (20) ermöglichen.

4. Haltevorrichtung (10) für die Wirbelsäule, nach Anspruch 3, dadurch gekennzeichnet, daß die genannten Schlitze (32,33), die auf jeweils einer Seite jeder Stützkante (31) vorgesehen sind, sich beim Einrasten des genannten vorläufig flxierenden Zwischcnringes (30) an dem genannten Montagestück (11) im wesentlichen senkrecht zu der Außenfläche des Verbindungsstabes (20) einstellen können, um eine optimale Auflage jeder bogenförmigen Stützkante (31) auf dem genannten Verbindungsstab (20) zu ermöglichen.

5. Haltevorrichtung (10) für die Wirbelsäule, nach einem der Ansprüche 1 bis 4, dadurch gekennzeichnet, daß an dem vorläufig fixierenden Zwischenring (30) zwei einrastende Zungen (35,36) ausgebildet sind, die symmetrisch zu der Achse X-X' zwischen den genannten Stützkanten (31) angeordnet sind, welche beiden einrastenden Zungen (35,36) sich bei der Anbringung des vorläufig fixierenden Zwischenringes (30) auf dem Montagestück (11) der Haltevorrichtung (10) elastisch zu verformen vermögen und sich gegen eine an dem Montagestück (11) vorgesehenen Kante (14) legen.

6. Haltevorrichtung (10) für die Wirbelsäule, nach einem der Ansprüche 2 bis 5, dadurch gekennzeichnet, daß der Sitz (12) des genannten Montagestücks (11) im oberen Abschnitt mit einem Innengewinde (16) versehen ist, das mit dem mit Außengewinde versehenen, in den genannten Sitz (12) zu schraubenden Verriegelungselement (13) zusammenwirken soll, wobei der vorläufig fixierendo Ring (30) dem genannten Verbindungsstab (20) in dem genannten Sitz (12) eine vorläufige Lage zu geben vermag, so daß das Innengewinde (16) für das Einschrauben des Verriegelungselements (13) freigehalten ist.

7. Montageeinrichtung für eine Haltevorrichtung (10) für die Wirbelsäule, nach einem der Ansprüche 1 bis 6, dadurch gekennzeichnet, daß sie eine erste Zange (110) aufweist, die zwischen ihren Backen (111,112) den Fuß des Montagestücks (11) der genannten Haltevorrichtung (10) für die Wirbelsäule einzuspannen vermag, sowie eine zweite Zange (120), die an einem Ende eine insgesamt U-förmige Klaue (121) aufweist, die rittlings auf den Verbindungsstab (20) gesetzt werden kann, um diesen dem Sitz (12) des Montagestücks (11) der Haltevorrichtung (10) gegenüberzustellen, sowie eine Hülse (122), die auf der genannten Klaue (121) verschiebbar ist und dazu dient, den vorläufig fixierenden Zwischenring (30) zu halten, um diesen durch elastische Verformung mit dem Montagestück (11) der Haltevorrichtung (10) zu verrasten und den genannten Verbindungsstab (20) vorläufig in den Sitz (12) einzuführen, wobei die erste Zange (110) und die zweite Zange (120) jeweils einen Anschlag (113, 123) aufweisen, welche Anschläge (113,123) dazu vorgesehen sind, sich bei dem Zusammenbau der Haltevorrichtung (10) für die Wirbelsäule so aneinander abzustützen, daß die durch, die zweite Zange (120) bei dem Verrasten durch elastische Verformung des vorläufig fixierenden Zwischenringes (30) mit dem Montagestück (11) der Haltevorrichtung (10) auf die Haltevorrichtung (10) ausgeübten Druckkräfte aufgefangen werden.
